# EUROPÄISCHE PATENTSCHRIFT

(11) **EP 0 956 502 B1**
(45) Veröffentlichungstag und Bekanntmachung des Hinweises auf die Patenterteilung: **24.10.2001**
(21) Anmeldenummer: 98904069.6
(22) Anmeldetag: 14.01.1998
(51) Int. Cl.: G01N 27/414

(54) **VERFAHREN ZUR BESTIMMUNG DER CHEMISCHEN REAKTIVITÄT, INSBESONDERE DER KORROSIVITÄT, VON ÖLIGER FLÜSSIGKEIT ODER VERUNREINIGUNGEN IN DIESER**
METHOD FOR DETERMINING CHEMICAL REACTIVITY, SPECIALLY CORROSIVENESS OF AN OILY LIQUID OR IMPURITIES THEREIN
PROCEDE POUR LA DETERMINATION DE LA REACTIVITE CHIMIQUE, NOTAMMENT DE LA CORROSIVITE D'UN LIQUIDE HUILEUX OU D'IMPURETES CONTENUES DANS CELUI-CI

(30) Priorität: 30.01.1997 DE 19703357
(43) Veröffentlichungstag der Anmeldung: 17.11.1999
(73) Patentinhaber: Conti Temic microelectronic GmbH, 90411 Nürnberg (DE)
(72) Erfinder: VOIGT, Hartmut, D-25560 Oldenborstel (DE); VOIGT, Norbert, D-86978 Hohenfurch (DE)
(74) Vertreter: Maute, Hans-Jürgen, Dipl.-Ing.
(86) Internationale Anmeldenummer: EP9800185
(87) Internationale Veröffentlichungsnummer: WO9834103

(56) Entgegenhaltungen:
- US-A- 4 322 680
- US-A- 4 589 970
- US-A- 4 851 104

## Beschreibung

Die Erfindung betrifft ein Verfahren zur Bestimmung der chemischen Reaktivität, insbesondere der Korrosivität, von öliger Flüssigkeit oder Verunreinigungen in dieser gemäß dem Patentanspruch 1.

Auch in öligen Flüssigkeiten treten beispielsweise aufgrund von Verunreinigungen Redoxreaktionen (Redox-Potential) zwischen der Lösung und metallischen Oberflächen und somit Spannungspotentiale auf. Die auftretenden Spannungspotentiale sind dabei recht gering. Während Spannungspotentiale in wäßrigen Lösungen, also Lösungen mit hohem Elekrolytanteil, durch herkömmliche amperiometrische oder potentiometrische Meßverfahren und einfache Bezugselektroden auch bei kleinen Meßwerten noch ermittelt werden können, ist dies für ölige Flüssigkeiten mit sehr schlechter Leitfähigkeit nicht oder nicht ohne erhebliche Nachteile möglich.

So ist für die Durchführung potentiometrischer Meßverfahren die Zugabe von Elektrolyten erforderlich, das Verfahren also nicht reagenzienfrei durchführbar. Ein solches Verfahren setzt aber eine Entnahme einer Probe voraus, da nach der Zugabe von Elektrolyten die Probe meist unbrauchbar wird. Eine kontinuierliche Messung, insbesondere in Tanks, wäre daher mittels dieses Verfahrens nicht oder nur mit erheblichem Aufwand möglich. Potentiometrische Verfahren werden bspw. genutzt, um den pH-Wert einer Lösung zu bestimmen, der jedoch in allen nichtwäßrigen Lösungen ohne Zugabe von Elektrolyten nicht definiert ist.

Im Stand der Technik ist auch bekannt, daß chemische Spannungspotentiale als Meßgröße für andere Größen, wie zum Beispiel die Korrosivität einer öligen Lösung signifikant sind. Korrosivität ist ein Summenparameter, der die chemische Oxydationsneigung von Lösungen mit metallischen Oberflächen beschreibt und sich ergibt aus dem pH-Wert der Lösung, dem Elektrolytanteil und somit abhängig von der Konzentration und Art von freien Ionen ist. So basiert ein amperiometrisches Verfahren zur Bestimmung der Korrosivität von Mineralölen auf entsprechenden Redox-Potentialen. Die Bestimmung der Korrosivität ermöglicht die Beurteilung der Qualität von Mineralölen, welche bspw. in der Kfz-Technik zum Korrosionsschutz eingesetzt werden. Hierbei wird zwischen zwei unterschiedlich beschaffenen Elektroden im Öl ein Konstantstrom geregelt. Der elektrische Leitwert der Lösung ist jedoch sehr störanfällig, da er stark von der räumlichen Anordnung der Elektroden und dem Mineralöl, insbesondere dem Füllstand abhängig ist und darüber hinaus temperaturabhängig ist. Nachteilig ist weiterhin die fortschreitende chemische Veränderung der Elektrodenoberflächen und die Verunreinigung des Mineralöls durch Reaktionsprodukte der Redox-Reaktion an den Elektroden.

Generell sind auch sogenannte ionensensitive Feldeffekttransistoren (ISFET) zur Messung spannungspotentialabhängiger Größen, bspw. des pH-Wertes, bekannt. Diese weisen eine pH-Wert-sensitive Gateoberfläche, z.B. aus Tantalpentoxid (Ta₂O₅), auf und werden durch eine Referenzspannung an der Bezugselektrode in der Lösung auf einen Arbeitspunkt gesteuert und die Leitfähigkeit des Feldeffektkanals zwischen dem Drain- und Sourcegebiet ausgewertet. Derartige Anordnungen sowie der Aufbau solcher ISFET wird in der Literatur bereits beschrieben (vgl. Sensors - A Comprehensive Survey, edited by Göpel, Hesse, Zemel, VCH Weinheim u.a. 1991, Vol.2, S. 474 ff. oder Hauptmann, Peter: Sensoren - Prinzipien und Anwendungen, Hanser Verlag München Wien, 1990, S. 119). pH-ISFET werden jedoch insbesondere zur pH-Wertmessung wäßriger Lösungen verwendet und dazu die Bezugselektrode mit einer vom pH-Wert bzw. der Ionenkonzentration unabhängigen Referenzspannung beaufschlagt.

Der Einsatz von ionensensitiven Feldeffekttransistoren in öligen Lösungen ist aus der Patentschrift JP 08015219 zur Ölverschmutzungsmessung bereits erwähnt, jedoch werden in dieser Schrift nitratsensitive Gateoberflächen vorgeschlagen, die nur zur Bestimmung des Nitratanteils verwendbar sind, welcher bei pflanzlichen Ölen von Interesse ist. Ein Zusammenhang zu Spannungspotentialmessungen ist nicht erkennbar. Mineralöle bspw. weisen darüber hinaus auch keine nennenswerten Nitratanteile auf noch sind Nitratanteile in besonderem Maße korrosivitätsbeeinflussend. Wiederum wird eine Referenzspannung an einer Bezugselektrode zur Messung verwendet. Nitratsensitive ISFETs lassen sich außerdem bisher nur mit einer Enzymgateschicht realiseren, deren Lebensdauer nur einige Wochen beträgt.

Neben der üblichen Bildung eines elektrischen Feldes zwischen unterschiedlichen chemischen und biochemischen Substanzen und dem Gate eines ISFET, bei entsprechender Wahl des Gatematerials, ist in der US 4,322,680 außerdem eine Sensitivität für Redox-Reaktionen innerhalb einer nicht näher definierten Substanz zu entnehmen. Die Redox-Reaktion findet auf der ISFET-Oberfläche selbst statt und das Spannungspotential entsteht am Gate selbst, wie dies auch bei allen gezeigten Beispielen der Sensitivität chemischer Substanzen der Fall ist. (vgl. US 4,322,680: Spalte 7, Z. 40 - 46 sowie Spalte 9, Z. 3- 8).

Die Redoxsensitivität der ISFET-Oberfläche ist jedoch auch für die angegebenen ISFET-Strukturen recht schwach. Dem Fachmann wird dabei eine chemisch inaktive Referenzelektrode nahegelegt. Neben Edelmetallen oder Edelmetalllegierungen werden für herkömmliche ISFET-Anwendungen, wie bspw. in der US 4,322,680, insbesondere vorzugsweise sogenannte Elektroden der 2. Art verwendet, bspw. eine Ag/AgCl-Elektrode, bestehend aus einem AgCl-beschichteten Silberdraht innerhalb einer 3 mol KCl-Lösung, deren elektrischer Kontakt zur Lösung durch eine Glasschliffmembran (sogenannter Stromschlüssel) sichergestellt wird, ohne daß sich im Inneren der Elektrode die Chloridlonenkonzentration ändert. Darüber hinaus wird über die externe Spannungsquelle ein elektrisches Feld in der ausreichend elektrolythaltigen Lösung erzeugt, daß das an der ISFET-Gateoberfläche entstehende Spannungspotential erhöht. Um die Beeinflussung des Feldeffektkanals zu verhindern, muß die externe Spannung möglichst konstant gehalten und chemische Reaktionen an der Referenzelektrode vermieden werden (vgl. US 4.322,680: Spalte 11, Z. 57 ff.). Das Referenzpotential an der Bezugselektrode bleibt bei Elektroden der 2. Art konstant, da die Chlorid-Ionenkonzentration nicht beeinflußt wird. Zum extern erzeugten elektrischen Feld (vgl. US 4,322,680: Fig. 2) addiert sich das an der Gateoberfläche entstehende Feld.

Aufgabe der Erfindung ist es daher, ein Verfahren zur Bestimmung der chemischen Reaktivität, insbesondere der Korrosivität, von einer öligen Flüssigkeit oder Verunreinigungen in dieser vorzustellen, welches einfach, störunanfällig, reagenzienfrei und kontinuierlich auch in Tanks angewendet werden kann.

Die Aufgabe ist durch die kennzeichnenden Merkmale des Patentanspruchs 1 gelöst. Vorteilhafte Weiterbildungen sind den Unteransprüchen zu entnehmen.

Der ISFET reagiert bereits auf geringe Spannungspotentiale zwischen der chemisch reaktiven Bezugselektrode und der öligen Flüssigkeit beziehungsweise Verunreinigungen in dieser. Das Spannungspotential entsteht also zwischen der Bezugselektrode und der Öllösung, nicht wie bei bisherigen ISFET-Anwendungen üblich am ionensensitiven Gate. Der ISFET wirkt somit in der Öllösung entgegen der bisherigen Ionensensivität wie ein hochohmiger passiver Spannungsmeßfühler, bei dem sich die Leitfähigkeit des Feldeffektkanals in Abhängigkeit von dem bei den chemischen Reaktionen an der Bezugselektrode entstehenden Spannungspotential ändert, während alle bekannten Anwendungen von ISFETs gerade von einer konstanten Spannung an der Bezugselektrode ausgingen. Durch eine elektrische Verbindung der Bezugselektrode wird an dem Source- oder dem Drainanschluß ein Bezugspotential geschaffen, so daß am Gatepotential das Spannungspotential als Feldeffekt auf den Feldeffektkanal einwirkt. Die Leitfähigkeit des Feldeffektkanals kann dann ausgewertet werden.

Dieses Verfahren erweist sich als äußerst vorteilhaft, da es reagenzienfrei ohne Zugabe von Elektrolyten arbeitet und bereits auf die geringen, auf natürliche Weise an metallischen Oberflächen auftretenden Spannungspotentiale reagiert und stromlos und damit ohne vermeidbare Rückstände in der öligen Flüssigkeit auswertet. Das Verfahren erweist sich als sehr störunempfindlich, da die Temperatur und die Leitfähigkeit des Mineralöls nahezu keinen Einfluß auf das Meßergebnis hat. Entgegen alle bekannten Verfahren ist es für Lösungen mit schlechter Leitfähigkeit anwendbar.

Gemäß Patentanspruch 2 wird vorgeschlagen, bei einem metallischen Tankgehäuse dieses als Bezugselektrode zu verwenden. Diese bevorzugte Weiterbildung bedarf keiner gesonderten Bezugselektrode, weist aufgrund der großen Oberfläche eine störunanfällige Charakteristik und eine besonders weitgehende Unabhängigkeit von der Füllhöhe der öligen Flüssigkeit auf. Sie ist einfach und kostengünstig zu realisieren. Sie ist außerdem für die kontinuierliche Qualitätskontrolle von öligen Lösungen in diesen Tanks äußerst sinnvoll, da die bei der Messung eventuell nachzuweisenden Redox-Reaktionen zwischen Tank und

Öllösung unabhängig von der Messung sind und auch kontinuierlich stattfinden.

Gateschichten aus Tantalpentoxid (Ta₂O₅) gemäß Patentanspruch 3 sind besonders empfindlich, aber auch sehr stabil und langlebig, was für die Langzeitanwendungen in Tanks, Motorölwannen ect. von wesentlicher Bedeutung ist. Die Ionen- bzw. pH-Wert-Sensitivität der Tantalpentoxid-Feldeffekttransistoren ist als solche nicht entscheidend, da die ISFETS in dieser Anwendung als passiver Meßfühler eingesetzt werden. In öligen Lösungen sind die Konzentrationen jedoch auch sehr gering. Höhere Konzentrationen könnten zu einer eher unerwünschten Querempfindlichkeit führen und den eigentlichen Meßeffekt überlagern.

ISFETs mit Gateschichten aus Siliziumnitrid (Si₃N₄) gemäß Patentanspruch 4 sind gegenüber solchen mit Tantalpentoxid deutlich preiswerter und weisen gleichzeitig noch sehr zufriedenstellende Empfindlichkeit und Stabilität auf.

Die Verwendung eines Constant Charge Mode (CCM)-Reglers gemäß Patentanspruch 5 erweist sich als besonders geeignet, da so der Feldeffekttransistor in einem konstanten Arbeitspunkt gehalten und die Verfälschung der Meßergebnisse durch die Nichtlinerarität der Kennlinie vermieden wird.

Die Bezugselektrode kann außerdem spannungsfrei auf Masse geschaltet werden, wodurch Polarisationseffekte an der Gateoberfläche vermieden werden.

Besonders hervorzuheben ist die vorteilhafte Verwendung des erfindungsgemäßen Verfahrens zur Beurteilung der Korrossivität von öligen Flüssigkeiten, insbesondere Mineralölen, gemäß Patentanspruch 7. Reichern sich durch Umwelteinflüsse und Verunreinigungen bspw. in Mineralölen korrosive Stoffe an, insbesondere Wasser und Säuren, so führt dies zu natürlichen Redox-Reaktionen mit metallischen Oberflächen, in deren Folge ein Spannungspotential an der Grenzfläche entsteht. Aufgrund des geringen Anteils von freien Elektrolyten im Mineralöl ist dabei der Effekt der pH-Wertmessung gering gegenüber der Abhängigkeit von der anliegenden Redox-Spannung. Das Spannungspotential ist durch das erfindungsgemäße Verfahren einfach, reagenzienfrei und stromlos erfaßbar, so daß es sich in besonders guter Weise auch für die kontinuierliche Beurteilung der Korrosivität eignet. Ihr Einsatz bietet sich daher insbesondere für Mineralöle in Motoren oder Hydraulikanlagen an und kann bspw. durch Signallampen die Verschlechterung des Korrosionsschutzes und einen fälligen Ölwechsel anzeigen.

Eine weitere vorteilhafte Verwendung des Verfahrens ist gemäß Patentanspruch 8 die Bestimmung des Wasseranteils in öligen Flüssigkeiten. So sind bspw. Bremsflüssigkeiten von öliger Konsistenz, weisen aber eine starke hydroskopische Wirkung auf, die dazu führt, daß sich die Bremsflüssigkeit mit Wasser anreichert. Dieses führt aber in zunehmenden Maße zur Beeinträchtigung der Bremsleistung. da bei den im Bremsmoment aufgrund von Reibungswärme auftretenden hohen Temperaturen die Wasseranteile verdampfen und so im Gegensatz zur Bremsflüssigkeit komprimierbar werden. Durch hydroreaktive Bezugselektroden, wie als Beispiel Silberchlorid, entsteht ein Spannungspotential, welches erfindungsgemäß mittels ISFET nachgewiesen werden kann.

Da Mineralöle häufig länger in metallischen Tanks gelagert oder beweglichen metallischen Geräteteilen als Schmier- und Korrosionsschutzmittel eingesetzt werden, ist eine besonders vorteilhafte Weiterbildung der Erfindung darin zu sehen, das erfindungsgemäße Verfahren kontinuierlich zur Qualitätskontrolle in diesen Tanks einzusetzen , da so frühzeitig eine Verschlechterung der Mineralölqualität erkannt und entsprechend neues Mineralöl oder neue Tanks verwendet werden können.

Erfindung wird nachfolgend anhand von Ausführungsbeispielen und Figuren näher erläutert.

Kurze Beschreibung der Figuren:
- Figur 1: Prinzipskizze des Verfahrens mit einer separaten Bezugselektrode
- Figur 2: Prinzipskizze des Verfahrens mit einer metallischen Ölwanne
- Figur 3: Prinzipskizze der CCM-Regelung
- Figur 4: Visualisierung der möglichen elektrischen Felder bei ISFETs

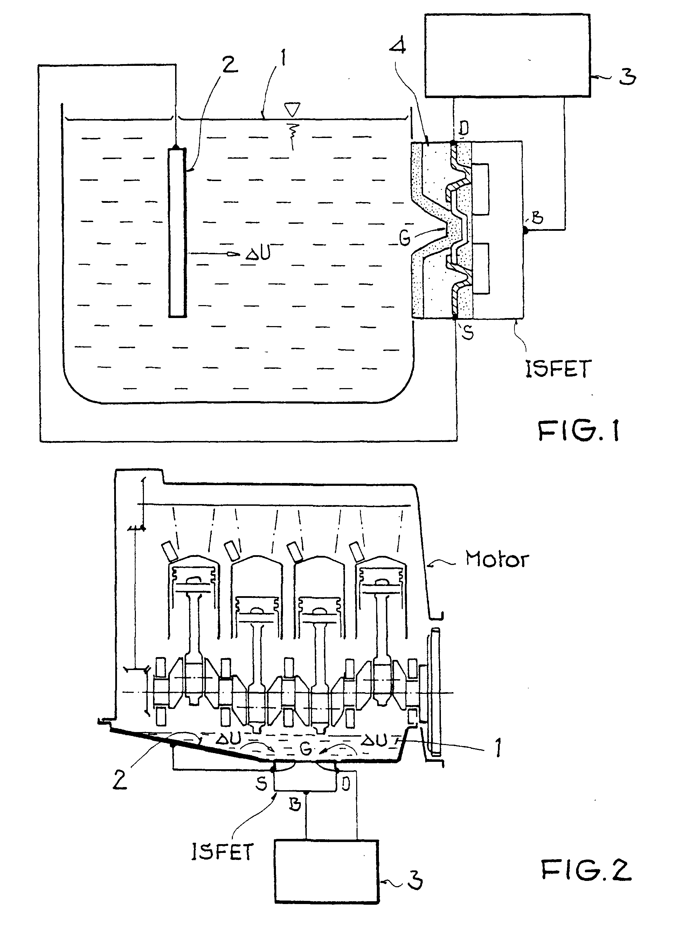

Die Figur 1 zeigt einen ionensensitiven Feldeffekttransistor (ISFET) in einer vereinfachten Schnittdarstellung mit einem Source-Anschluß S; einem Drain-Anschluß D, einem Bulk-Anschluß B und einer Gateoberfläche G, die ionensensitiv ist und von der öligen Flüssigkeit (1) benetzt wird. Drain- und Source-Gebiet des ISFET sind dabei von der öligen Flüssigkeit durch Isolationsflächen 4 getrennt. Es wird ein pH-sensitiver ISFET mit einer Tantalpentoxidschicht (Ta₂O₅) verwendet. In diesem Ausführungsbeispiel ist die in der öligen Flüssigkeit befindliche Bezugselektrode 2 mit dem Source-Anschluß verbunden, wobei bei entsprechend anderer Polung der Versorgungsspannung oder des CCM-Reglers 3, der in diesem Fall die Versorgungsspannung bereitstellt, auch ein drainseitiger Anschluß der Bezugselektrode denkbar ist. An der Bezugselektrode 2 tritt durch Redox-Reaktionen ein Spannungspotential ΔU auf, was zu einer Änderung der Leitfähigkeit des Feldeffektkanals führt, was wiederum mit einer in den CCM-Regler 3 integrierten Auswerteschaltung ermittelt werden kann.

Figur 2 zeigt die vorteilhafte Verwendung zur Bestimmung der Korrosivität in einer metallischen Ölwanne, beispielsweise in einem Verbrennungsmotor, als Bezugselektrode 2. Die metallische Ölwanne ist aus Gußeisen und weist dabei ein auf natürliche Weise sich ausbildendes Spannungspotential ΔU aufgrund von Redox-Reaktionen mit Verunreinigungen, insbesondere Säuren und Wasser, im Mineralöl 1 auf, die sich durch Umwelteinflüsse im Mineralöl 1 ansammeln. Der ISFET ist mit der ionensensitiven Gateoberfläche G am Boden der Ölwanne angeordnet und kommt dort mit der Öllösung 1 und ihren Verunreinigungen in Berührung.

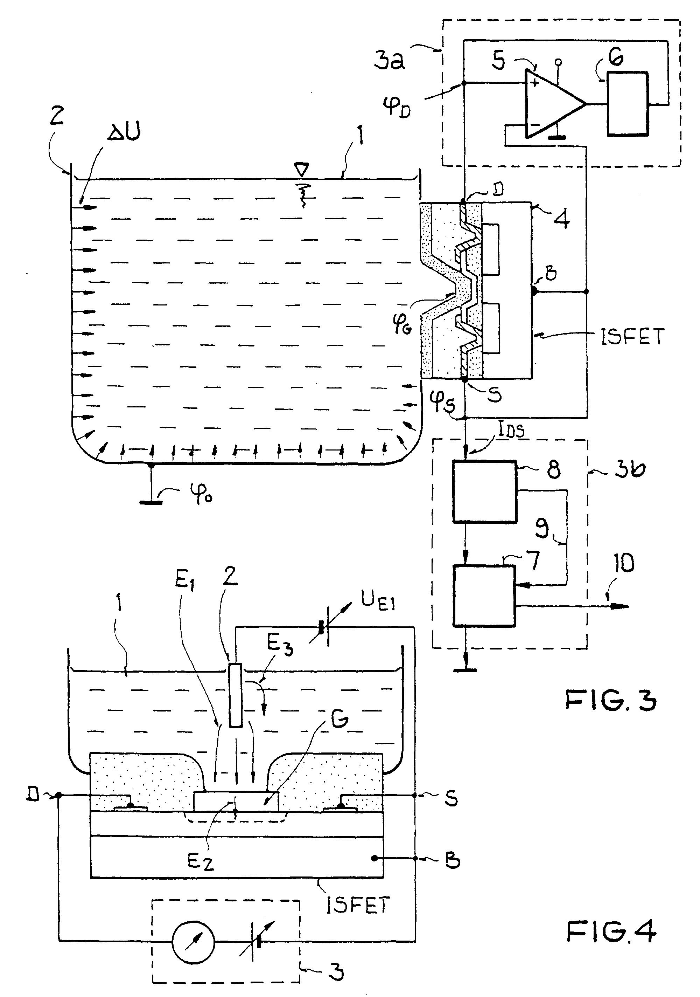

In Figur 3 wird ein Ausführungsbeispiel des erfindungsgemäßen Verfahrens gezeigt, bei dem die CCM-Regelung 3, zur besseren Darstellung bestehend aus 2 Teilen 3a und 3b, so angepaßt ist, daß die Ölwanne als Bezugselektrode 2 auf Massepotential ϕ₀ geschaltet und über dieses mit dem ISFET verbunden ist. Dies ist insbesondere für Anwendungen in Kraftfahrzeugen sehr vorteilhaft, da im allgemeinen alle Gehäuseteile auf Masse geschaltet sind und somit eine elektrische Isolation entfallen kann.

Entsteht durch Redox-Reaktionen zwischen der Ölwanne als Bezugselektrode 2 und der Öllösung 1 ein Spannungspotential ΔU, so breitet sich ein entsprechendes elektrisches Feld in der Öllösung 1 aus. Es erhöht sich entsprechend das Potential an der Gateoberfläche ϕ_{G} um ΔU, wodurch sich der Widerstand des Feldeffektkanals verändert, da zunächst das Sourcepotential ϕₛ konstant ist und damit die Gate-Source-Spannung sich somit verändert. In diesem Zusammenhang soll nun die CCM-Regelung erläutert werden. Da ISFETs wie alle Transistoren eine nichtlineare Kennlinie aufweisen, ist es für quantitative meßtechnische Anwendungen sinnvoll, den ISFET in einem konstanten Arbeitspunkt zu betreiben, wobei die in Fig. 1 und 2 gezeigten Schaltungsvarianten eines CCM-Reglers 3 für weniger hohe Anforderungen, insbesondere qualitative Aussagen, durchaus ausreichen.

Zur Konstanthaltung des Arbeitspunktes muß einerseits die Drain-Source-Spannung konstant gehalten werden. Dazu ist der Teil 3a vorgesehen. Der Komparator 5 ermittelt die Differenz der ihm zugeführten Potentiale ϕ_{S} und ϕ_{D}, also die Drain-Source-Spannung. Der Regler 6 führt das Drainpotential ϕ_{D} entsprechend einer voreingestellten Solldifferenz nach. Das Substrat des ISFET wird über den Bulk-Anschluß B, wie bei Feldeffekttransistoren üblich auf Source S geschaltet.

Außerdem ist der Strom zwischen Drain D und Source S konstantzuhalten. Will man gleichzeitig die Drain-Source-Spannung konstanthalten, kann bei sich verändertem Gatepotential ϕ_{G} und folglich sich verändertem Feldeffektkanalwiderstand anstelle dessen das Sourcepotential ϕₛ nachgeführt werden, da über die Gate-Source-Spannung ja der Feldeffekt bestimmt ist. Dazu ist der Teil 3b der CCM-Regelung vorgesehen. Er besteht aus einer Stromerkennung 8, der der Strom durch den ISFET zugeführt wird und eines mittels des Stromänderungssignals 9 ansteuerbaren Reglers 7 zur Steuerung des Sourcepotentials ϕₛ.

Bildet sich also aufgrund von Redox-Reaktionen zwischen der Ölwanne als Bezugselektrode 2 und der Öllösung 1 ein Spannungspotential ΔU ändert sich das Potential an der Gateoberfläche ϕ_{G}, verändert sich kurzfristig der Widerstand des Feldeffektkanals. Der Strom durch den ISFET verändert sich, was in der Stromerkennung 8 erkannt wird. Der Regler 7 verändert daraufhin das Sourcepotential ϕ_{S} derart, daß die Gate-Source-Spannung auf einen voreingestellten Wert zurückgeführt wird. Der Strom nimmt somit wieder seinen Sollwert an. Gleichzeitig mit der Anpassung des Sourcepotentials ϕ_{S} wird durch den Teil 3a das Drainpotential ϕ_{D} entsprechend nachgeführt,so daß auch die Drain-Source-Spannung wieder im Arbeitspunkt ist. Das Maß der Änderung der eingetretenen Änderung der Leitfähigkeit des Feldeffektkanals, der durch die Gegensteuerung mittels des Sourcepotentials ϕ_{S} wieder aufgehoben wurde, wird als Maß der Reaktivität, insbesondere der Korrosivität, des Mineralöls 1 verwendet. Dazu wird die Gegensteuerung des Sourcepotentials ϕ_{S} als Meßsignal 10 zur weiteren Verarbeitung abgeführt. Dieses kann nachfolgend bspw. mit Schwellweten verglichen und die Zeit bis zu einem erforderlichen Ölwechsel angezeigt werden.

Figur 4 visualisiert nochmals die möglichen elektrischen Felder bei ISFETs. Wird zur Einstellung des Arbeitspunktes eine Spannung U_{E1} an die Bezugselektrode 2 geschaltet, so bildet sich in der Lösung 1 ein elektrisches Feld E1 zum Gate hin aus. Für herkömmliche ISFET-Anwendungen der ionensensitiven Gateoberflächen G wird E1 konstant gehalten. Es ist insbesondere der im Vergleich zu E1 jedoch deutlich schwächere Feldeffekt E2, der sich auf E1 auflagert, am Gate G selbst entscheidend. Demgegenüber ist bei dem erfindungsgemäßen Verfahren der ISFET ein passiver Meßfühler. Der Feldeffekt E2 tritt nicht oder nur in geringem Maße auf. Er ist nicht erforderlich, aufgrund seiner geringen Wirkung aber meist auch nicht störend. Entscheidend ist das durch das Spannungspotential ΔU hervorgerufene Feld E3, was bisher durch edelmetallische Bezugselektroden nicht auftrat. Bei einer auf Masse geschalteten Bezugselektrode, also U_{E1}=0, entfallen auch Polarisationseffekte an der Gateoberfläche G.

Die chemisch reaktive Bezugselektrode 2 ist so zu wählen, daß einerseits ein Spannungspotential ΔU bei der nachzuweisenden Reaktivität auftritt. So kommen grundsätzlich alle unedlen Metalle oder Metallverbindungen mit Redox-Eigenschaften in Frage, insbesondere auch Eisen. Andererseits ist für eine kontinuierliche Messung der natürliche Verschleiß der Bezugselektrode 2 gering zu halten, so daß für Langzeitanwendungen sehr unedle Metalle, wie bspw. Magnesium, als ungeeignet erscheinen. Für kurzzeitige labortechnische Verwendungen des Verfahrens ist der Verschleiß und Austausch der Bezugselektrode 2 unproblematisch.

## Patentansprüche

1. Verfahren zur Bestimmung der chemischen Reaktivität, insbesondere der Korrosivität, von öliger Flüssigkeit oder Verunreinigungen in dieser,
a) indem eine chemisch reaktive Bezugselektrode mit der öligen Flüssigkeit in Berührung gebracht wird,
b) ein Feldeffekttransistor mit einer ionensensitiven Gate-Oberfläche (ISFET) in der öligen Flüssigkeit angeordnet wird, wobei der Feldeffekttransistor einen Drain- und einen Source-Anschluß sowie zwischen diesen einen Feldeffektkanal aufweist,
c) die chemisch reaktive Bezugselektrode elektrisch mit einem der Anschlüsse des Feldeffekttransistors, dem Source- oder dem Drain-Anschluß, verbunden ist und
d) die Änderung der Leitfähigkeit des Feldeffektkanals als Maß der Reaktivität verwendet wird.

2. Verfahren nach Anspruch 1, **dadurch gekennzeichnet, daß** als chemisch reaktive Bezugselektrode ein metallisches Gehäuse eines Tanks verwendet wird.

3. Verfahren nach Anspruch 1 oder 2, **dadurch gekennzeichnet, daß** der ionensensitive Feldeffekttransistor mit einer Tantalpentoxidgateschicht (Ta₂O₅) ausgeführt wird.

4. Verfahren nach Anspruch 1 oder 2, **dadurch gekennzeichnet, daß** der pH-sensitiver Feldeffekttransistor mit einer Siliziumnitridgateschicht (Si₃N₄) ausgeführt wird.

5. Verfahren nach einem der voranstehenden Ansprüche, **dadurch gekennzeichnet, daß** der Feldeffekttransistor mittels eines Constant Charge Mode (CCM)-Reglers mit einem konstanten Drain-Source-Strom betrieben wird.

6. Verfahren nach einem der voranstehenden Ansprüche, **dadurch gekennzeichnet, daß** die chemisch reaktive Bezugselektrode auf die elektrische Masse geschaltet und über Masse mit dem mit dem einem der Anschlüsse des Feldeffekttransistors verbunden ist.

7. Verwendung des Verfahrens nach den voranstehenden Patentansprüchen zur Beurteilung der Korrosivität von öligen Lösungen, indem eine Bezugselektrode in der öligen Lösungen verwendet wird, die zu Korrosion hervorrufenden Verunreinigungen der öligen Lösung chemisch reaktiv ist, und die Änderung der Leitfähigkeit des Feldeffektkanals als Maß der Korrosivität verwendet wird.

8. Verwendung des Verfahrens nach den Patentansprüchen 1 bis 6 zur Bestimmung des Wasseranteils in öligen Lösungen, insbesondere in hydroskopischer Bremsflüssigkeit, indem eine Bezugselektrode mit einer hydroreaktiven Oberfläche, bspw. aus Silberchlorid verwendet wird.

9. Verwendung des Verfahrens nach den Patentansprüchen 1 bis 6 zur kontinuierlichen Qualitätskontrolle von Mineralölen in Metalltanks, insbesondere in Motorölwannen.

## Claims

1. A method of determining the chemical reactivity and especially the corrosiveness of an oily liquid or impurities therein,
a) wherein a chemically reactive reference electrode is placed in contact with the oily liquid,
b) a field effect transistor having an ion sensitive gate surface (ISFET) is disposed in the oily liquid, whereby the field effect transistor comprises a drain and a source terminal together with a field effect channel therebetween,
c) the chemically reactive reference electrode is electrically connected to one of the terminals of the field effect transistor, to the source or to the drain terminal and
d) the alteration in the conductivity of the field effect channel is used as a measure for the reactivity.

2. A method in accordance with Claim 1, **characterised in that** a metallic housing of a tank is used as the chemically reactive reference electrode.

3. A method in accordance with Claim 1 or 2, **characterised in that** the ion sensitive field effect transistor is provided with a tantalum pentoxide gate layer (Ta₂O₅).

4. A method in accordance with Claim 1 or 2, **characterised in that** the pH sensitive field effect transistor is provided with a silicon nitride gate layer (Si₃N₄).

5. A method in accordance with any of the preceding Claims, **characterised in that** the field effect transistor is operated with a constant drain-source current by means of a Constant Charge Mode (CCM) regulator.

6. A method in accordance with any of the preceding Claims, **characterised in that** the chemically reactive reference electrode is connected to the electrical earth and is connected to the [sic] to one of the terminals of the field effect transistor via earth.

7. The use of the method in accordance with any of the preceding Claims, for assessing the corrosiveness of oily solutions wherein a reference electrode, which is chemically reactive to impurities in the oily solution causing corrosion, is used in the oily solutions and the alteration in the conductivity of the field effect channel is used as a measure for the corrosiveness.

8. The use of the method in accordance with any of Claims 1 to 6, for determining the water content of oily solutions, especially in a hydroscopic brake fluid, wherein a reference electrode having a hydro-reactive surface, e.g. of silver chloride, is used

9. The use of the method in accordance with any of Claims 1 to 6 for continuously checking the quality of mineral oils in metal tanks, especially in engine oil sumps.

## Revendications

1. Procédé de détermination de la réactivité chimique, en particulier de la corrosivité, d'un liquide huileux ou d'impuretés qui y sont contenues, dans lequel
e) on met une électrode de référence chimiquement réactive en contact avec le liquide huileux,
f) on dispose dans le liquide huileux un transistor à effet de champ muni d'une surface de grille sensible aux ions (ISFET), le transistor à effet de champ comprenant une connexion de drain et une connexion de source et entre les deux un canal d'effet de champ,
g) l'électrode de référence chimiquement réactive est reliée électriquement à l'une des connexions du transistor à effet de champ, la connexion de source ou la connexion de drain, et
h) on utilise comme mesure de la réactivité la variation de la conductivité du canal d'effet de champ.

2. Procédé selon la revendication 1, **caractérisé en ce que** l'on utilise comme électrode de référence chimiquement réactive une carcasse métallique d'un réservoir.

3. Procédé selon la revendication 1 ou 2, **caractérisé en ce que** le transistor à effet de champ sensible aux ions est muni d'une grille revêtue de pentoxyde de tantale (Ta₂O₅).

4. Procédé selon la revendication 1 ou 2, **caractérisé en ce que** le transistor à effet de champ sensible au pH est muni d'une grille revêtue de nitrure de silicium (Si₃N₄).

5. Procédé selon l'une des revendications précédentes, **caractérisé en ce que** le transistor à effet de champ fonctionne par l'intermédiaire d'un régulateur à mode de charge constante (CCM) avec un courant drain-source constant.

6. Procédé selon l'une des revendications précédentes, **caractérisé en ce que** l'électrode de référence chimiquement réactive est connectée à la masse électrique et est reliée par l'intermédiaire de la masse à l'une des connexions du transistor à effet de champ.

7. Application du procédé selon l'une des revendications précédentes pour l'estimation de la corrosivité de solutions huileuses, selon laquelle on utilise dans les solutions huileuses une électrode de référence qui réagit chimiquement avec les impuretés de la solution huileuse responsables de la corrosion, et on utilise la variation de la conductivité du canal d'effet de champ comme mesure de la corrosivité.

8. Application du procédé selon les revendications 1 à 6 pour déterminer la teneur en eau de solutions huileuses, en particulier d'un liquide de frein hygroscopique, dans laquelle on utilise une électrode de référence ayant une surface réagissant avec l'eau, par exemple en chlorure d'argent.

9. Application du procédé selon les revendications 1 à 6 pour le contrôle de qualité continu d'huiles minérales dans des réservoirs métalliques, en particulier dans des carters à huile de moteur.
